# EUROPÄISCHE PATENTSCHRIFT

(11) **EP 3 503 951 B1**
(45) Veröffentlichungstag und Bekanntmachung des Hinweises auf die Patenterteilung: **13.04.2022**
(21) Anmeldenummer: 17758449.7
(22) Anmeldetag: 10.08.2017
(51) Int. Cl.: A61M 15/00

(54) **VORRICHTUNG ZUM INHALIEREN PULVERFÖRMIGER SUBSTANZEN**
DEVICE FOR INHALING POWDERY SUBSTANCES
DISPOSITIF POUR L'INHALATION DE SUBSTANCES PULVÉRULENTES

(30) Priorität: 25.08.2016 DE 202016104666 U; 20.01.2017 DE 202017100316 U; 20.04.2017 DE 202017102334 U
(43) Veröffentlichungstag der Anmeldung: 03.07.2019
(73) Patentinhaber: Von Schuckmann, Alfred, 47627 Kevelaer (DE)
(72) Erfinder: Von Schuckmann, Alfred, 47627 Kevelaer (DE)
(74) Vertreter: Müller, Enno
(86) Internationale Anmeldenummer: PCT/EP2017/070342
(87) Internationale Veröffentlichungsnummer: WO 2018/036836

(56) Entgegenhaltungen:
- WO-A1-2012/047181
- WO-A1-2013/095311
- WO-A1-2015/128789
- GB-A- 2 407 042
- GB-A- 2 439 205
- US-A1- 2008 105 256
- US-A1- 2013 255 679

## Beschreibung

### Gebiet der Technik

Die Erfindung betrifft eine Vorrichtung gemäß dem beigefügten Anspruch 1. Eine Vorrichtung zum Inhalieren pulverförmiger Substanzen enthalten in Kapseln, mit einem Gehäuse, einer Kapselaufnahme, einem Verschlussdeckel und einem Mundstück, wobei eine zum Überfangen zugleich des Mundstücks, des Verschlussdeckels und der Kapselaufnahme ausgebildete Verschlusskappe vorgesehen ist, wobei weiter die Verschlusskappe, das Mundstück und der Verschlussdeckel verschwenkbar anscharniert sind, wobei zwei unterschiedliche, aber gleichgerichtet verlaufende Verschwenkachsen ausgebildet sind, eine erste Verschwenkachse und eine zweite Verschwenkachse, die, bezogen auf eine Seitenansicht, in welcher sich geometrische Achsen der Verschwenkachsen punktförmig abbilden, nebeneinander und auf einer selben Seite der Kapselaufnahme angeordnet sind, wobei weiter in einem Benutzungszustand der Vorrichtung die erste Verschwenkachse näher an der Kapselaufnahme angeordnet ist, der Verschlussdeckel und das Mundstück um die erste Verschwenkachse verschwenkbar sind und allein die Verschlusskappe um die zweite Verschwenkachse verschwenkbar ist.

### Stand der Technik

Eine derartige Vorrichtung ist zunächst aus der WO 2012/047182 A2 und der WO 2012/047181 A1 bekannt. Hierbei weist das Mundstück zwei in der genannten Seitenansicht, wenn eine Aufnahmerichtung für die Kapselaufnahme einer Vertikalen entspricht, untereinander angeordnete Verschwenkachsen auf. An der insoweit oberen Verschwenkachse ist der Verschlussdeckel angelenkt und an der insoweit unteren Verschwenkachse sind das Mundstück und die Kapselaufnahme zusammen an dem Gehäuse angelenkt. Eine der Verschwenkachsen ist fest an dem Gehäuse ausgebildet und die andere um diese gehäusefeste Schwenkachse mit der Kapselaufnahme und dem Mundstück verschwenkbar.

Zum Stand der Technik ist weiter auf die WO 2015/128789 A1 zu verweisen. Bei der hieraus bekannten Vorrichtung zum Inhalieren pulverförmiger Substanzen sind ebenfalls zwei Schwenkachsen vorgesehen, die in der genannten Seitenansicht und in einer Ausrichtung, bei welcher die Aufnahmerichtung der Kapselaufnahme vertikal verläuft, nebeneinander angeordnet sind. An der insoweit äußeren Verschwenkachse sind um dieselbe Achse verschwenkbar die Verschlusskappe und das Mundstück angeordnet und an der inneren, näher zu der Kapselaufnahme angeordneten Verschwenkachse ist lediglich der Verschlussdeckel, der hier zusammengefasst mit der Kapselaufnahme ausgebildet und verschwenkbar ist, angeordnet.

Schließlich ist es aus der US 2013/255679 A1 bekannt, bei einer derartigen Vorrichtung eine an dem Gehäuse integriert ausgebildete Schwenkachse vorzusehen, um welche die Verschlusskappe schwenkend bewegbar ist, jedoch ein dem Verschlussdeckel gleichzusetzendes Teil an einem Einsteckteil zum Einstecken in das Gehäuse vorzusehen. An dem Einsteckteil, im Bereich des Verschlussdeckels, ist das Mundstück verschwenkbar angelenkt.

### Zusammenfassung der Erfindung

Ausgehend von dem dargelegten Stand der Technik liegt der Erfindung die Aufgabenstellung zugrunde, eine Vorrichtung zum Inhalieren pulverförmiger Substanzen, wie sie eingangs angegeben wurde, derart weiterzubilden, dass bei günstiger Handhabung durch einen Benutzer eine gute Zugänglichkeit der Teile im aufgeschwenkten Zustand gegeben ist.

Diese Aufgabe ist beim Gegenstand des Anspruchs 1 gelöst, wobei darauf abgestellt ist, dass die Verschlusskappe, das Mundstück und der Verschlussdeckel jeweils um eine fest an dem Gehäuse angebrachte Verschwenkachse verschwenkbar anscharniert sind und beide Verschwenkachsen relativ zueinander unbeweglich an dem Gehäuse ausgebildet sind. Somit sind beide Verschwenkachsen fest an dem Gehäuse angebracht.

Es ist auch bevorzugt, dass nur zwei Verschwenkachsen an der Vorrichtung ausgebildet sind. In einer Weiterbildung können aber auch drei Verschwenkachsen vorgesehen sein, wobei die Verschlusskappe und der Verschlussdeckel jeweils unmittelbar an einer Verschwenkachse angelenkt sind und das Mundstück an einer an dem Verschlussdeckel ausgebildeten Verschwenkachse verschwenkbar angelenkt ist. Die Verschwenkachsen, um welche jeweils für sich die Verschlusskappe und der Verschlussdeckel verschwenkbar sind, sind entsprechend unterschiedliche Verschwenkachsen. Die weitere, dritte Verschwenkachse kann zusammen mit dem Verschlussdeckel verschwenkbar vorgesehen sein.

Die Kapselaufnahme umfasst bevorzugt auch eine Handhabe, die weiter bevorzugt schieberartig beweglich ist, und Nadeln, mit welchen eine in die Kapselaufnahme eingelegte Kapsel durch Einstechen öffenbar ist. Die Nadeln sind bevorzugt zur gemeinsamen Bewegung mit der Handhabe verbunden.

In weiter bevorzugter Ausgestaltung ist vorgesehen, dass das Mundstück und die Verschlusskappe bezogen auf die genannte Seitenansicht, in wel-(Weiter auf Seite 4, oben, der ursprünglich eingereichten Unterlagen) eher sich die geometrischen Achsen der Verschwenkachsen punktförmig abbilden, sich in einer am weitesten geöffneten Stellung bezüglich ihrer Außenkontur ohne Überdeckung zueinander erstrecken. Für den Nutzer ist in der weitesten geöffneten Stellung, der weitesten Schwenkstellung der Verschlusskappe und des Mundstückes, insbesondere das Mundstück vollständig sichtbar. Es liegt keine Einschachtelung des Mundstückes in die geöffnete Verschlusskappe vor.

Das Mundstück und der Verschlussdeckel sind bevorzugt miteinander verrastbar ausgebildet. Somit kann bei einem üblichen Aufschwenken des Mundstückes zugleich auch der Verschlussdeckel mit aufgeschwenkt werden. Soweit der Verschlussdeckel relativ verschwenkbar zu einer auch verschwenkbar angeordneten Kapselaufnahme ausgebildet ist, kann auch vorgesehen sein, dass der Verschlussdeckel auch mit der Kapselaufnahme verrastbar ist. Hierbei kann die Rastwirkung zu dem Mundstück bevorzugt stärker ausgebildet sein als eine Rastwirkung zu der Kapselaufnahme. In einem solchen Fall wird durch ein Aufschwenken des Mundstückes, zum Einlegen etwa einer neuen Kapsel, nicht zugleich auch die Kapselaufnahme aufgeschwenkt. Insbesondere wenn nach einem Aufschwenken des Mundstückes die Kapsel durch den nicht aufgeschwenkten Verschlussdeckel ohne Weiteres eingelegt werden kann, der Verschlussdeckel also nicht, wie grundsätzlich möglich, etwa mit einem Sieb versehen ist, ist es bevorzugt, dass die Rastwirkung des Verschlussdeckels zu der Kapselaufnahme größer ist als zu dem Mundstück. Wenn aber, wie weiter möglich und in vielen Fällen auch bevorzugt, zwar ein entsprechendes Sieb vorgesehen ist, dieses aber mit dem Mundstück verbunden ist, ist es bevorzugt, dass die Rastwirkung des Verschlussdeckels zu der Kapselaufnahme größer ist als zu dem Mundstück, so dass der Benutzer nur das Mundstück aufschwenkt, um eine neue Kapsel einlegen zu können.

Der Verschlussdeckel kann auch mit der Kapselaufnahme einstückig oder quasi einstückig (betrieblich nicht leicht oder ohne weiteres lösbar) ausgebildet sein. Dann führt ein benutzerseitiges Aufschwenken des Verschlussdeckels regelmäßig auch zu einem Aufschwenken der Kapselaufnahme.

Hierzu kann aber auch eine eigenständige Verrastbarkeit der Kapselaufnahme mit dem Gehäuse vorgesehen sein. Die Gesamtheit aus Verschlussdeckel und Kapselaufnahme kann nur über eine Verrastbarkeit der Kapselaufnahme mit dem Gehäuse oder nur über eine Verrastbarkeit des Verschlussdeckels mit dem Gehäuse verrastbar sein. In einem solchen Fall kann eine weitere Verrastbarkeit zwischen dem Verschlussdeckel und der Kapselaufnahme ausgebildet sein. Durch eine unterschiedliche Einstellung der Öffnungskraft zwischen dem Verschlussdeckel und der Kapselaufnahme kann eingestellt werden, ob mit Hochschwenken des Verschlussdeckels zugleich auch die Kapselaufnahme mit verschwenkt oder die Kapselaufnahme regelmäßig im Gehäuse verbleibt und nur bei Einwirkung sogleich auf die Kapselaufnahme die dann stärkere Öffnungskraft zur Lösung der Verrastung zwischen der Kapselaufnahme und dem Gehäuse aufgebracht werden kann, um den Verschlussdeckel und die Kapselaufnahme gemeinsam zu verschwenken.

In weiterer Ausgestaltung kann der Verschlussdeckel und/oder das Mundstück mit dem Gehäuse oder im Falle des Mundstücks alternativ mit dem Verschlussdeckel steckverbunden sein. Die Steckverbindung kann sich allein oder zumindest bevorzugt beziehen auf die Festlegung des Verschlussdeckels und/oder des Mundstücks im Bereich der jeweiligen Schwenkachse. So kann gemäß einer möglichen Ausgestaltung erst unter Erreichen der Steckverbindung zwischen dem Verschlussdeckel und/oder dem Mundstück und dem Gehäuse oder im Falle des Mundstückes zwischen dem Mundstück und dem Verschlussdeckel die Schwenkachse gebildet sein. Die miteinander steckverbindbaren Teile weisen hierzu bevorzugt jeweils Abschnitte der im Nutzungszustand der Vorrichtung gegebenen Schwenkachse auf.

Die mögliche Steckverbindung erweist sich insbesondere als fertigungstechnisch von Vorteil. Die jeweiligen Achsabschnitte der miteinander steckverbindbaren Teile können bei einer bevorzugten Ausgestaltung der Teile als Kunststoffspritzteile im Zuge des Kunststoffspritzverfahrens unmittelbar mit ausgebildet werden.

So kann der Verschlussdeckel angeformte Achszapfen aufweisen, zur Steckaufnahme in Steckausformungen des Gehäuses oder das Gehäuse angeformte Achszapfen aufweisen, zur Steckaufnahme in Steckausformungen des Verschlussdeckels. Die Steckausformungen können mit Bezug auf einen Querschnitt quer zur Ausrichtung der geometrischen Schwenkachse klammerartig den Achszapfen übergreifend ausgebildet sein, wobei bevorzugt Bereiche der Steckausformungen ein mit Bezug auf die geometrische Achse radiales, flexibles Ausweichen ermöglichen, um so die Steck-Rastverbindung nach Überlaufen und Einfangen des Achszapfens herzustellen.

In einer möglichen Ausgestaltung ist so der Verschlussdeckel mit dem Gehäuse steckverbunden und das Mundstück mit dem Verschlussdeckel.

Es ergibt sich bezüglich der Steckausformungen im Zuge des Einsteckvorganges eine Verformungstendenz. Diese kann in einer möglichen Ausgestaltung insbesondere im Nutzungszustand der Vorrichtung, d.h. insbesondere in der Verschlussstellung des Verschlussdeckels unterbunden sein. Hierzu können die Steckausformungen des Gehäuses im Nutzungszustand der Vorrichtung entgegen der beim Einsteckvorgang sich ergebenden Verformungstendenz von einem Bereich des Verschlussdeckels umfasst oder tangiert sein. Insbesondere in der Nutzungsstellung bspw. des Verschlussdeckels ist so die Steckverbindung nicht aufhebbar. Die diesbezüglichen Steckausformungen können nicht zur Freigabe der aufgenommenen Achszapfen ausweichen, da diese insbesondere wandungsaußenseitig von einem Bereich des Verschlussdeckels gehindert sind.

Zur Erlangung der Steckverbindung insbesondere zwischen Verschlussdeckel und Gehäuse ist somit in einer bevorzugten Ausgestaltung eine Ausrichtung des Verschlussdeckels relativ zu dem Gehäuse nötig, die nicht dem Nutzungszustand entspricht.

Der Achszapfen kann in einem Querschnitt mit einer kreisringförmigen Wandung ausgebildet sein. So kann der Achszapfen als Hohlzapfen gebildet sein. Eine solche Ausgestaltung bietet Vorteile insbesondere hinsichtlich der Erlangung der Steckverbindung in Zusammenwirkung mit der den Achszapfen aufnehmenden bzw. umfassenden Steckausformung.

Der Verschlussdeckel kann eine Steckausformung für eine Verschwenkachse des Mundstücks aufweisen. Alternativ kann das Mundstück eine Steckausformung für einen Achszapfen des Verschlussdeckels aufweisen. So sind Verschlussdeckel und Mundstück miteinander steckverbindbar, weiter insbesondere zur Ausbildung einer gemeinsamen Schwenkachse, um welche das Mundstück und, bevorzugt unabhängig davon, der Verschlussdeckel relativ zu dem Gehäuse aus einer Nutzungsstellung heraus bzw. in diese hinein verschwenkbar sind.

Die Steckrichtungen zum Erreichen der Steckverbindungen zwischen dem Verschlussdeckel und dem Gehäuse einerseits und dem Mundstück und dem Verschlussdeckel andererseits können, projiziert in eine Ebene senkrecht zu der geometrischen Schwenkachse des Verschlussdeckels, in Umfangsrichtung zu der geometrischen Schwenkachse einen spitzen oder rechten Winkel zueinander einschließen. So kann in einer möglichen Ausgestaltung die Steckrichtung zum Erreichen der Steckverbindung zwischen dem Verschlussteil und dem Gehäuse etwa senkrecht zu einer Flächenerstreckung des Verschlussdeckels (bezogen auf die Nutzungsstellung) gewählt sein, während die Steckrichtung zum Erreichen der Steckverbindung zwischen dem Mundstück und dem Verschlussdeckel bspw. im Wesentlichen senkrecht zu einer Querschnittsfläche des Verschlussdeckels, in welcher Querschnittsfläche sich die geometrische Schwenkachse als Linie darstellt, gewählt sein kann.

Der Winkel zwischen den beiden Steckrichtungen kann bspw. 30 Grad bis hin zu 90 Grad betragen, so weiter bspw. auch 45 oder 65 Grad, darüber hinaus aber auch weniger als 30 Grad, bspw. bis hin zu 20 oder 15 Grad.

### Kurze Beschreibung der Zeichnungen

Nachstehend ist die Erfindung anhand der beigefügten Zeichnung erläutert, die aber lediglich Ausführungsbeispiele darstellt. Ein Teil, das nur bezogen auf eines der Ausführungsbeispiele erläutert ist und bei einem weiteren Ausführungsbeispiel aufgrund der dort herausgestellten Besonderheit nicht durch ein anderes Teil ersetzt ist, ist damit auch für dieses weitere Ausführungsbeispiel als jedenfalls mögliches vorhandenes Teil beschrieben. Auf der Zeichnung zeigt:
- Fig. 1: eine Querschnittsansicht der Vorrichtung zum Inhalieren im geschlossenen Zustand, eine erste Ausführungsform betreffend;
- Fig. 2: eine ungeschnittene Seitenansicht der Vorrichtung gemäß Figur 1 im geöffneten Zustand;
- Fig. 3: eine weitere Ausführungsform, bei welcher der Verschlussdeckel und die Verschlusskappe um eine gemeinsame zweite Verschwenkachse an einer gemeinsamen, zweiten Verschwenkachse verschwenkbar sind und das Mundstück an einer an dem Verschlussdeckel ausgebildeten ersten Verschwenkachse verschwenkbar ist, im geöffneten Zustand;
- Fig. 4: eine weitere Ausführungsform, bei welcher entsprechend Figur 1 der Verschlussdeckel und die Verschlusskappe an zwei unterschiedlichen, gehäusefesten Verschwenkachsen anscharniert sind und das Mundstück um eine weitere an dem Verschlussdeckel ausgebildete Verschwenkachse verschwenkbar ist, im geschlossenen Zustand;
- Fig. 5: eine Darstellung der Vorrichtung gemäß Figur 4, mit aufgeschwenktem Mundstück und Verschlusskappe;
- Fig. 6: eine Darstellung gemäß Figur 5, in welcher auch der Verschlussdeckel aufgeschwenkt ist;
- Fig. 7-9: eine Darstellung unterschiedlicher Siebvarianten;
- Fig. 10: die Vorrichtung in einer weiteren Ausführungsform im geschlossenen Zustand;
- Fig. 11: die Vorrichtung gemäß Figur 10 in partiell geschnittener perspektivischer Darstellung, den geöffneten Zustand der Vorrichtung betreffend;
- Fig. 12: die Vorrichtung im geschlossenen Zustand gemäß Figur 1 in einer Querschnittsansicht;
- Fig. 13: die Herausvergrößerung des Bereichs XIII in Figur 11, jedoch mit einem in Nutzungsstellung verschwenktem Verschlussdeckel;
- Fig. 14: den Schnitt gemäß der Linie XIV-XIV in Figur 13;
- Fig. 15: den Verschlussdeckel und das Mundstück in perspektivischer Einzeldarstellung;
- Fig. 16: den Schnitt gemäß der Linie XVI-XVI in Figur 13.

### Beschreibung der Ausführungsformen

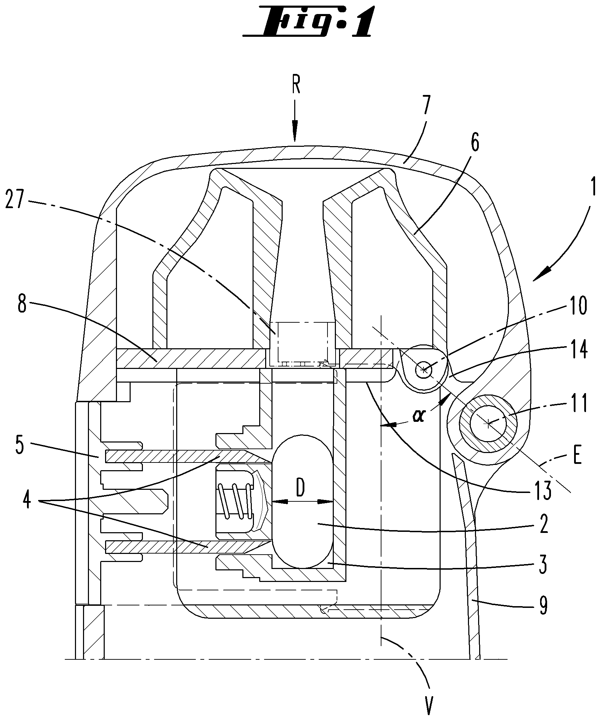

Dargestellt und beschrieben ist mit Bezug zu Figur 1 eine Vorrichtung 1 zum Inhalieren pulverförmiger Substanzen. Die pulverförmigen Substanzen sind hierbei in Kapseln 2 enthalten, die in einer Kapselaufnahme 3 der Vorrichtung aufnehmbar sind. Mittels Nadeln 4 können die Kapseln 2 durch Betätigung an einer Handhabe 5 durchstochen werden. Beim nachfolgenden Saugen eines Nutzers an einem Mundstück 6 kann er die in den Kapseln 2 enthaltene pharmazeutische Substanz hierdurch inhalieren.

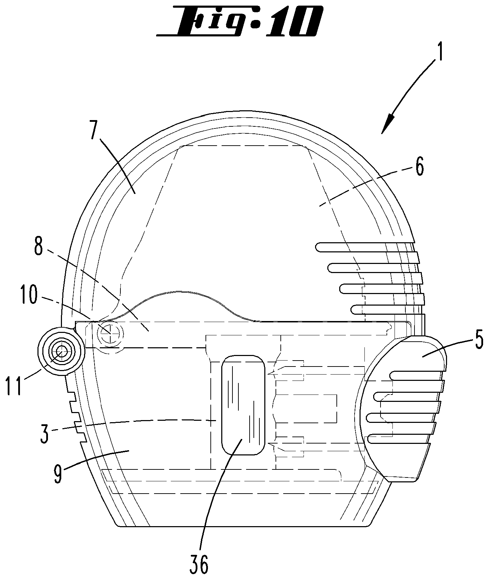

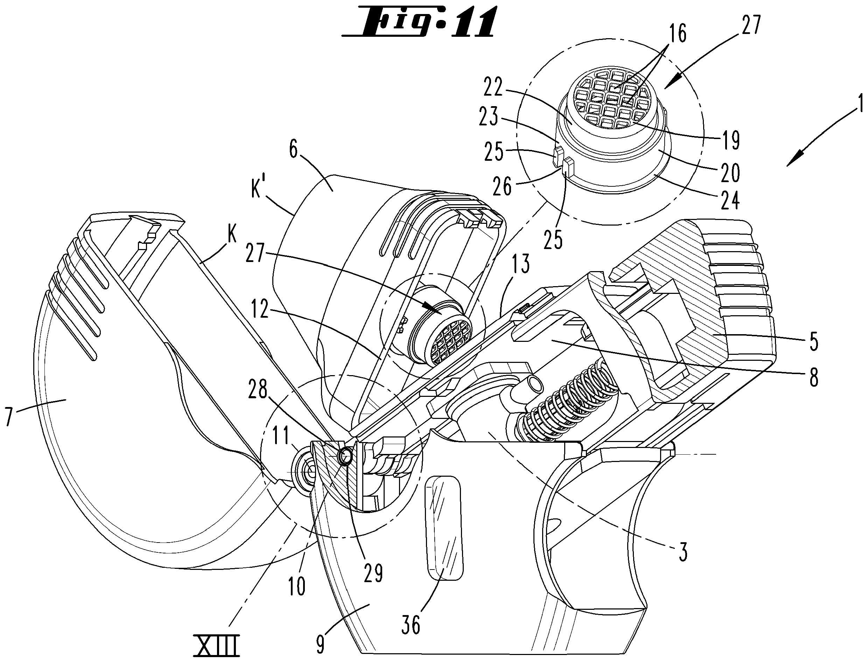

Durch ein ggf. gehäuseseitig vorgesehenes Sichtfenster 36, wie dies beispielhaft in den Figuren 10 und 11 dargestellt ist, kann kontrolliert werden, ob eine Kapsel 2 in der Kapselaufnahme 3 einliegt.

Die Vorrichtung 1 weist weiter eine Verschlusskappe 7 auf, die im geschlossenen Zustand der Vorrichtung, wie in Figur 1 dargestellt, das Mundstück 6 überfängt. Unterhalb des Mundstücks weist die Vorrichtung weiterhin einen Verschlussdeckel 8 auf. Der Verschlussdeckel 8 kann mit der Kapselaufnahme 3 fest, jedenfalls drehfest, verbunden ausgebildet sein.

Die Verschlusskappe 7, das Mundstück 6 und der Verschlussdeckel 8 sind an einem - unteren - Gehäuse 9 verschwenkbar anscharniert. Es sind hierzu zwei unterschiedliche Verschwenkachsen ausgebildet, eine erste Verschwenkachse 10 und eine zweite Verschwenkachse 11.

Die Verschwenkachsen 10 und 11 sind bevorzugt - zumindest über einen Teil der Breite der Vorrichtung - als reale Achsteile oder Achszapfen, die in entsprechenden Aufnahmen, insbesondere ösenartigen Aufnahmen, zur Schwenkbeweglichkeit aufgenommen sind, ausgebildet. Die Aufnahmen sind hierbei bevorzugt in dem Gehäuse 9 oder auch bezüglich eines der Teile Mundstück 6 oder Verschlussdeckel 8 an dem anderen Teil ausgebildet.

Die Achsen 10 und 11 weisen weiter jeweils eine in der Zeichnung durch ein Kreuz angedeutete geometrische Achse auf, die durch den Mittelpunkt der hier dargestellten realen Achsen 10 und 11 verläuft.

In der genannten Querschnittsansicht bspw. der Figur 1, die einer Seitenansicht entspricht, in welcher sich die genannten geometrischen Achsen der Verschwenkachsen 10, 11 punktförmig abbilden, liegen die Achsen 10 und 11 nebeneinander. Sie liegen - bezogen auf die genannte Darstellung, in welcher eine Einführrichtung R für die Kapselaufnahme einer Vertikalen entspricht - nicht notwendig in einer rechtwinklig zu der genannten Einführrichtung R verlaufenden Ebene, die hier einer Horizontalen entsprechen würde. Vielmehr sind sie in vertikaler Richtung bevorzugt unterschiedlich angeordnet, wobei weiter bevorzugt die äußere, zweite Verschwenkachse 11 tiefer angeordnet ist als die innere, erste Verschwenkachse 10. Eine Ebene E durch die Verschwenkachsen 10, 11 in Figur 1 schließt also in der sich hier ergebenden Abbildung als Linie einen spitzen Winkel α mit einer Vertikalen V ein. Die Vertikale V entspricht in ihrer Ausrichtung in den Figuren der Einführrichtung R. Der Winkel α kann bspw. zwischen 0 und 30° liegen. Die Unterschiedlichkeit der Anordnung in Vertikalrichtung ist aber bevorzugt gering, entspricht etwa hinsichtlich des vertikalen Abstandes (bezogen auf einen vertikalen Abstand zwischen jeweils einer durch die erste Verschwenkachse 10 und die zweite Verschwenkachse 11 gezogenen Horizontalen) bevorzugt nur einer Hälfte bis einem Zwanzigstel einer größten freien horizontalen Abmessung D der Kapselaufnahme 3.

Der Verschlussdeckel 8 und das Mundstück 6 sind zusammen um die erste Verschwenkachse 10 verschwenkbar und die Verschlusskappe 7 ist allein um die zweite Verschwenkachse 11 verschwenkbar.

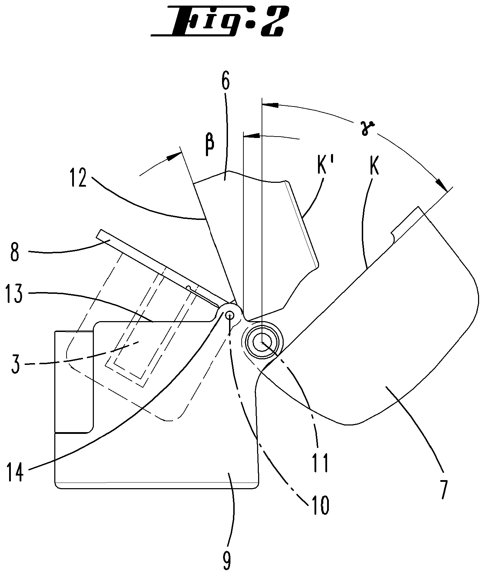

Eine aufgeschwenkte Stellung der Teile ist in Figur 2 ersichtlich.

Hierbei ist bevorzugt wie auch dargestellt gegeben, dass das Mundstück 6 und die Verschlusskappe 7 sich in einer am weitesten geöffneten Stellung bezüglich ihrer Außenkontur K bzw. K' ohne Überdeckung zueinander erstrecken. Die Aufschwenkstellung des Mundstückes 6 kann hierbei derart gegeben sein, dass eine dem Verschlussdeckel 8 zugeordnete Randkante 12, die weiter bevorzugt etwa durchgehend geradlinig verläuft, im maximal aufgeschwenkten Zustand entsprechend einer Vertikalen verläuft (bezogen auf die Darstellung gemäß Figur 1) bzw. einen ersten spitzen Winkel β mit der Vertikalen einschließt, etwa einen Winkel zwischen 1 und 30°.

Dem gegenüber schließt ersichtlich und bevorzugt die entsprechende Randkante des Verschlussdeckels 7 in der genannten Öffnungsstellung mit einer Vertikalen einen zweiten, bevorzugt größeren, spitzen Winkel γ zwischen 0 bis 45° ein. Während der erste spitze Winkel β zu beiden Seiten der Vertikalen in der maximalen Verschwenk-Öffnungsstellung des Mundstücks 6 gegeben sein kann, ist der zweite spitze Winkel γ des Verschlussdeckels 7 mit der Vertikalen im maximalen Öffnungszustand des Verschlussdeckels 7 nur bezüglich der der Kapselaufnahme abgewandten Seite der Vertikalen gegeben.

Die erste Verschwenkachse 10 kann weiter, wie beim Ausführungsbeispiel dargestellt, bezüglich einer Oberkante 13 des Gehäuses 9 in einer nach oben vorragenden Lagerausformung 14 aufgenommen sein. Hiermit lässt sich noch günstiger die Aufspreizung in der Öffnungsstellung gemäß Figur 2 oder Figur 11 erreichen.

Das Mundstück 6 und der Verschlussdeckel 8 können miteinander verrastbar sein. Sie können in einer miteinander verrasteten Stellung zusammen verschwenkbar sein. Weiter können der Verschlussdeckel 8 und die Kapselaufnahme 3, wie insbesondere in einer der Ausgestaltungen wie weiter vorne auch erläutert, zu einer gemeinsamen Verschwenkung miteinander verbunden sein.

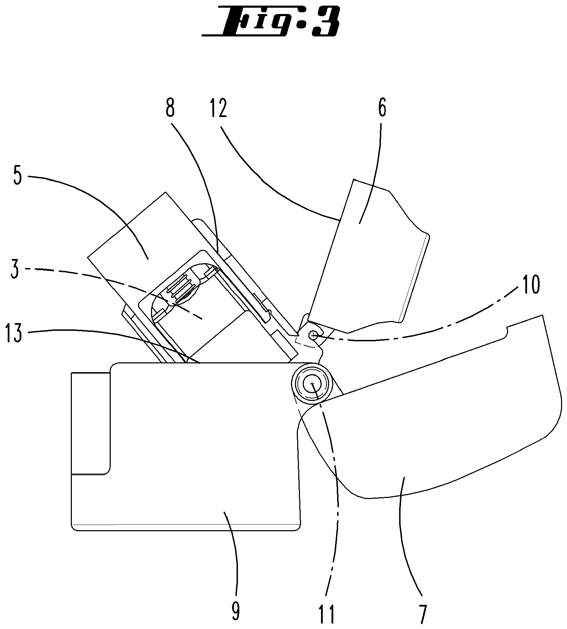

Mit Bezug zu Figur 3 ist eine weitere Ausführungsform dargestellt, bei welcher die zweite Verschwenkachse 11 eine gemeinsame Verschwenkachse für den Verschlussdeckel 8 und die Verschlusskappe 7 ist, während die erste Verschwenkachse 10 an einer Oberseite des Verschlussdeckels 8 angebracht ist und um diese erste Verschwenkachse 10 allein das Mundstück 6 verschwenkbar ist. Entsprechend ergibt sich hiermit, dass, insofern auch in Übereinstimmung mit der ersten Ausführungsform, das Mundstück 6 und der Verschlussdeckel 8 auch in zusammengefasster, bevorzugt verrasteter Form zusammen verschwenkbar sind. Im Fall der Ausführungsform der Figur 3 ist die gemeinsame Verschwenkbarkeit des Verschlussdeckels und des Mundstückes 6 um die zweite Verschwenkachse 11 gegeben. Im Weiteren ergibt sich auch, dass im vollständig aufgeschwenkten Zustand, wie er bezüglich jedenfalls des Mundstückes 6 und der Verschlusskappe 7, bevorzugt aber auch hinsichtlich des Verschlussdeckels 8 in Figur 3 dargestellt ist, auch hierbei die konturenmäßige vollständige Trennung zwischen der Verschlusskappe 7 und dem Mundstück 8 ergeben kann.

Hinsichtlich weiterer Einzelheiten hinsichtlich der konturenmäßigen Trennung wird auch auf die Beschreibung des ersten Ausführungsbeispiels verwiesen.

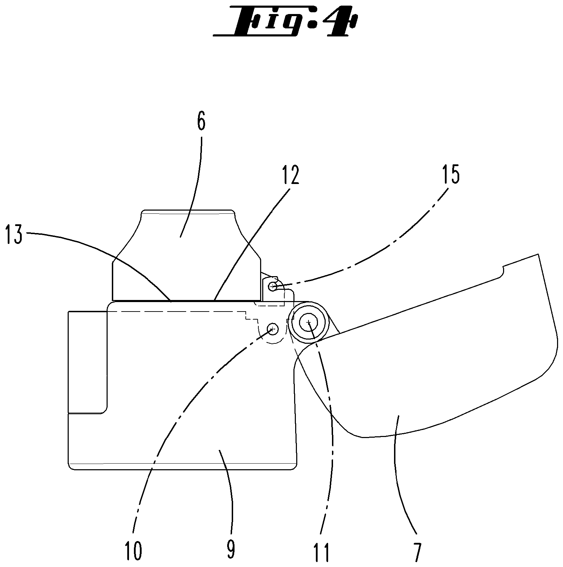

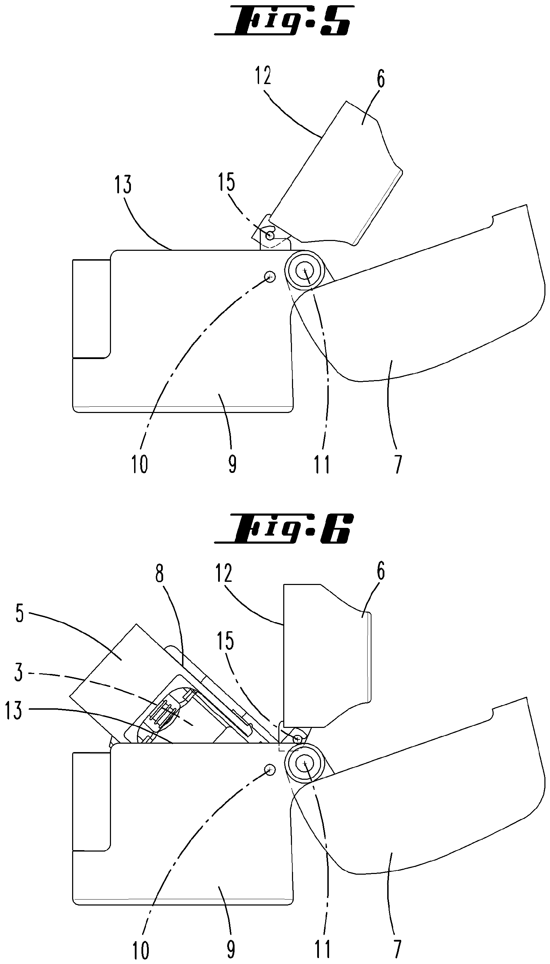

Mit Bezug zu den Figuren 5 bis 6 ist nochmals eine weitere Ausführungsform dargestellt, die hinsichtlich der ersten Schwenkachse 10 und der zweiten Schwenkachse 11 der ersten Ausführungsform der Figuren 1 bis 2 entspricht. Bei dieser Ausführungsform kann jedoch entgegen der Darstellungen der Ausführungsformen der Figuren 1 und 2 die erste Schwenkachse 10 tiefer, bevorzugt geringfügig tiefer, angeordnet sein als die zweite Verschwenkachse 11. Diese Ausgestaltung lässt sich andererseits aber auch bei der Ausführungsform der Figuren 1 und 2 verwirklichen. Hinsichtlich des vertikalen Abstandes bei der Ausführungsform der Figuren 4 bis 6 der ersten und zweiten Schwenkachse ergeben sich umgekehrt gleiche Verhältnisse wie bei der ersten Ausführungsform beschrieben.

Im Weiteren ist auch hier der Verschlussdeckel 8 mit einer gesonderten, in dem Fall allerdings dritten Verschwenkachse 15 für das Mundstück 6 ausgebildet.

Von Besonderheit ist weiter, dass das Mundstück 6 aus der Aufnahme für die dritte Verschwenkachse 15 durch Entrasten entfernt werden kann. Gleiches ist grundsätzlich auch in allen hier beschriebenen Ausführungsformen für die erste und/oder zweite Schwenkachse zusätzlich möglich.

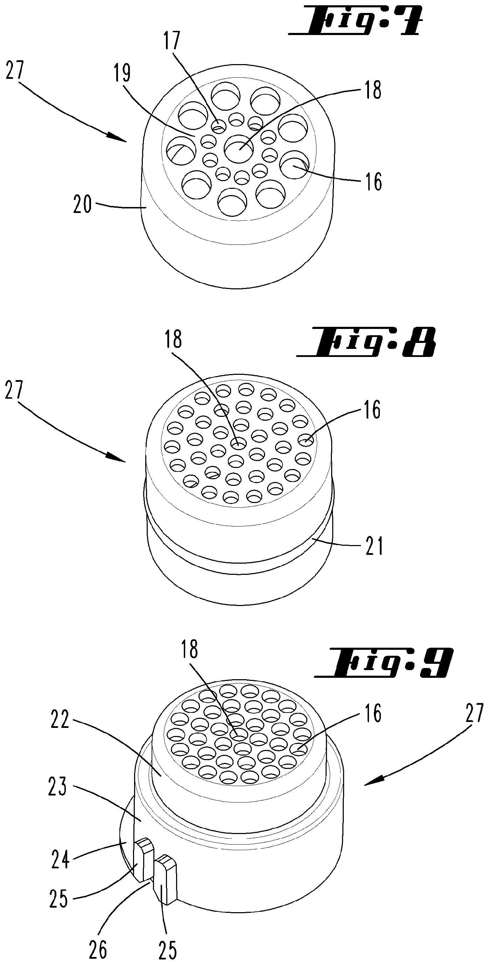

Mit Bezug zu den Figuren 7 bis 9 (weiter auch in Figur 11) sind unterschiedliche Ausführungsformen für ein Sieb 15 dargestellt. Ein Sieb 15 kann in dem Mundstück bezüglich aller Ausführungsformen in der in Figur 1 angedeuteten Weise angeordnet sein.

Bei der Ausführungsform der Figur 7 weist das Sieb 15 zwei koaxiale Kränze von Löchern 16 und 17 auf. Hierbei sind die Löcher 16, die bevorzugt außenseitig zu den Löchern 17 angeordnet sind, mit einem größeren Durchmesser versehen. Der Durchmesser eines Loches 17 entspricht bevorzugt zwei Drittel oder weniger, bis hin etwa zu einem Viertel, eines Durchmessers des Loches 16.

Weiter ist ein zentrales einzelnes Loch 18 vorgesehen. Das Loch 18 wiederum besitzt bevorzugt einen größeren Durchmesser als die Löcher 17. Weiter bevorzugt entspricht der Durchmesser des Loches 18 dem eines Loches 16.

Bei der Ausführungsform der Figur 8 sind derartige Löcher bezüglich dreier konzentrischer Kreislinien vorgesehen, wobei weiter bevorzugt die Löcher 16 der Ausführungsform der Figur 8 sämtlich einen gleichen Durchmesser aufweisen. Einschließlich auch eines mittigen Loches 18.

Die Ausführungsform der Figur 9 entspricht hinsichtlich der Löcher bevorzugt der Ausführungsform der Figur 8.

Auch können die Löcher 16 gemäß der in Figur 11 dargestellten Ausführungsform rechteckige, bis hin zu quadratische, weiter bevorzugt gleichgroße Grundrisse aufweisen.

Ein derartiges Siebteil 27 ist weiter mit einem Topfboden 19 und einer Topfwandung 20 ausgebildet.

Bei der Ausführungsform der Figur 8 ist die Topfwandung 20 mit einem umlaufenden Wulst 21 versehen, etwa in der Mitte ihrer Wandungshöhe. Der Wulst 21 verläuft außenseitig an der Topfwandung 20. Hierdurch lässt sich eine günstige Einpressung und Halterung in dem Mundstück erreichen.

Bei der Ausführungsform der Figur 9 ist die Topfwandung gestuft ausgebildet. Ausgehend von dem Topfboden schließt sich ein erster Topfwandungsabschnitt 22 und ein zweiter Topfwandungsabschnitt 23 an. Der Durchmesser des zweiten Topfwandungsabschnittes 23 ist größer als der Durchmesser des ersten Topfwandungsabschnittes 22.

Weiter kann die Topfwandung 20 oder 22, 23 an ihrem dem Topfboden 19 abgewandten freien Ende zumindest über einen Teil ihres Umfangs einen nach außen kragenden Abschnitt 24 aufweisen (siehe auch Figur 11). Der Abschnitt 24 dient zur günstigen Haltung im Einbauzustand. Weiter kann an der Topfwandung auch etwa mittels vertikal verlaufender Rippen 25 eine Einführnut 26 ausgebildet sein. Dies ist insbesondere günstig im Hinblick auf einen gerichteten Einbau des Siebes in das Mundstück. Mundstückseitig kann dann ein entsprechender Vorsprung ausgebildet sein, der in die Nut 26 eingreift.

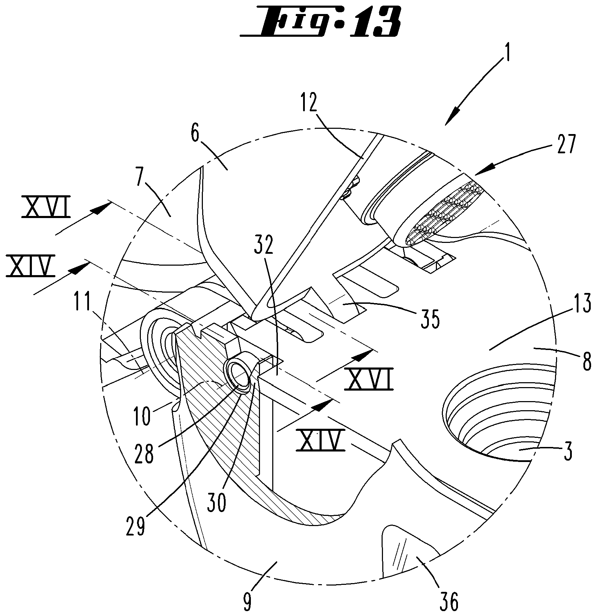

Die Figuren 10 bis 16 zeigen eine weitere Ausführungsform der Vorrichtung 1, bei welcher der Verschlussdeckel 8 mit dem Gehäuse 9 und das Mundstück 6 mit dem Verschlussdeckel 8 steckverbunden sind. Verschlussdeckel 8 und Mundstück 6 sind um eine gemeinsame Verschwenkachse 10 schwenkbeweglich gehaltert, wobei die Schwenkbeweglichkeit des Mundstückes 6 um die Verschwenkachse 10 unabhängig sein kann von der Schwenkbeweglichkeit des Verschlussdeckels 8 um dieselbe Verschwenkachse 10. Darüber hinaus kann aber auch ein gemeinsames Verschwenken vorgegeben sein, bspw. bei einer weiteren Verrastung, wie beschrieben, die ggf. durch den Benutzer aufhebbar sein kann.

Die Steckverbindung ist in dem dargestellten Ausführungsbeispiel gegeben durch Achszapfen und damit steckverbindbaren Steckausformungen.

So sind an dem Verschlussdeckel 8 bevorzugt jeweils etwa endseitig einer Längsrandkante des Verschlussdeckels 8 nach außen weisende und frei abragende Achszapfen 28 angeformt, zur Steckaufnahme in Steckausformungen 29 des Gehäuses 9. Die Steckausformungen 29 sind in Form von gehäusewandungsinnenseitig angeordneten taschenförmigen Nuten ausgebildet, die zur Oberkante 13 des Gehäuses 9 hin und nach innen, d.h. bezüglich der Verschwenkachse 10 aufeinander zu weisend offen gestaltet sind.

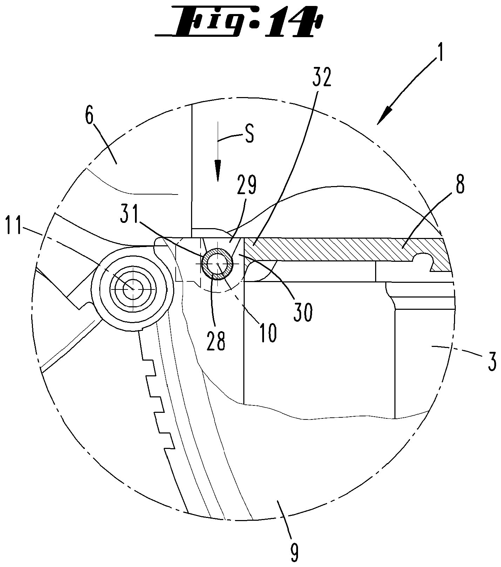

Mit Bezug auf einen Querschnitt, bei welchem sich die geometrische Verschwenkachse 10 punktförmig darstellt (vgl. Figur 14) ist eine gabelförmige Ausgestaltung der Steckausformung 29 zu erkennen, mit einem Nutengrund, der in diesem Querschnitt kreisabschnittförmig und durchmessermäßig an den Achszapfen 28 angepasst ausgebildet ist. Die Kreisabschnittform erstreckt sich über einen Umfang von mehr als 180 Grad, sodass in der Steckverbindungsstellung der aufgenommene Achszapfen 28 unverlierbar eingefangen ist.

Ausgehend von dem kreisabschnittförmigen Nutgrund erstreckt sich die Steckausformung 29 in dem Querschnitt trichterartig sich erweiternd bis zur Oberkante 13.

Insbesondere der der Verschlusskappen-Verschwenkachse 11 abgewandte Steckausformungsschenkel 30 kann geeigneter Weise im Zuge der Steckverbindung, d.h. im Zuge des Eintauchens des Achszapfens 28 in die Steckausformungen 29 zur Erlangung der Hintergriffstellung gemäß Figur 14 mit Bezug zu der Verschwenkachse 10 radial ausweichen.

Auch kann, wie dargestellt, der Achszapfen 28 eine kreisringförmige Wandung 31 bilden. Diese ist bevorzugt, insbesondere aufgrund der gegebenen Eigenschaften des bevorzugten Kunststoffmaterials, elastisch nachgebbar, was weiter zu einem günstigen Steckvorgang führt.

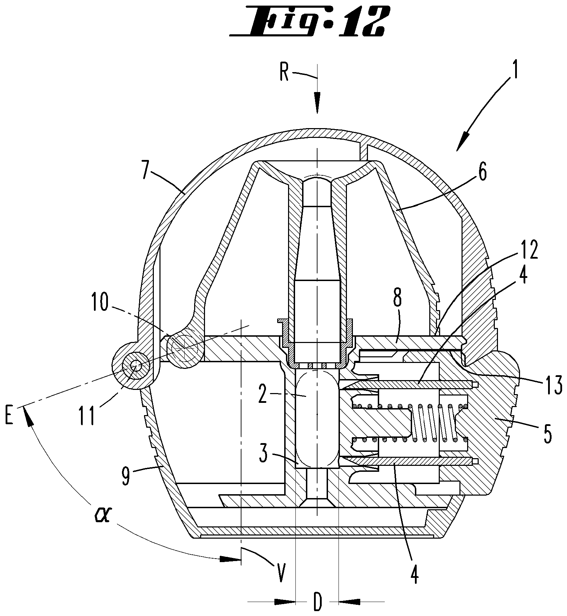

Bevorzugt wird der Verschlussdeckel 8 in einer Schwenkstellung desselben eingerastet, in welcher sich der Verschlussdeckel 8 außerhalb des Nutzungszustands gemäß bspw. den Figuren 12 bis 14 befindet, weiter bspw. in einer mit Bezug auf einen Querschnitt um etwa 90 Grad hierzu aufgeschwenkten Stellung.

In der abgeschwenkten Stellung, entsprechend der üblichen Nutzungsstellung des Verschlussdeckels 8, ist die Steckausformung 29 des Gehäuses 9, insbesondere der jeweilige Steckausformungsschenkel 30 entgegen einer beim Einsteckvorgang sich ergebenden Verformungstendenz von einem benachbarten, ggf. in der Nutzungsstellung sogar an dem Steckausformungsschenkel 30 anliegenden Bereich 32 des Verschlussdeckels 8 umfasst, womit die Steckverbindung zwischen Verschlussdeckel 8 und Gehäuse 9 im Bereich der Verschwenkachse 10 in der Nutzungsstellung gesichert ist. Der Steckausformungsschenkel 30 ist so in der Nutzungsstellung an einer Ausweichbewegung gehindert.

Zwischen den die nach axial außen weisenden Achszapfen 28 tragenden Verschlussdeckelabschnitten sind zu diesen Abschnitten, wie auch zueinander in Achsrichtung beabstandet, zwei gabelartige Steckausformungen 33 ausgebildet. Im Wesentlichen ergibt sich hierbei eine Ausgestaltung etwa gleich den Steckausformungen 29 des Gehäuses 9, wenngleich eine Öffnung der Steckausformungen 33 in Richtung auf die verschlusskappenseitige Verschwenkachse 11 gegeben ist.

In die Steckausformungen 33 sind entsprechend positionierte Achszapfen 34 des Mundstückes 6 einsteckbar, die an einem entsprechend positionierten Ausleger 35 angeformt sind.

In der Steckverbindungsstellung sind die Steckausformungen 29 des Gehäuses 9, die Achszapfen 28 des Verschlussdeckels 8, wie auch dessen Steckausformungen 33 und die Achszapfen 34 des Mundstückes 6 gemeinsam mittig von der geometrischen Verschwenkachse 10 durchsetzt.

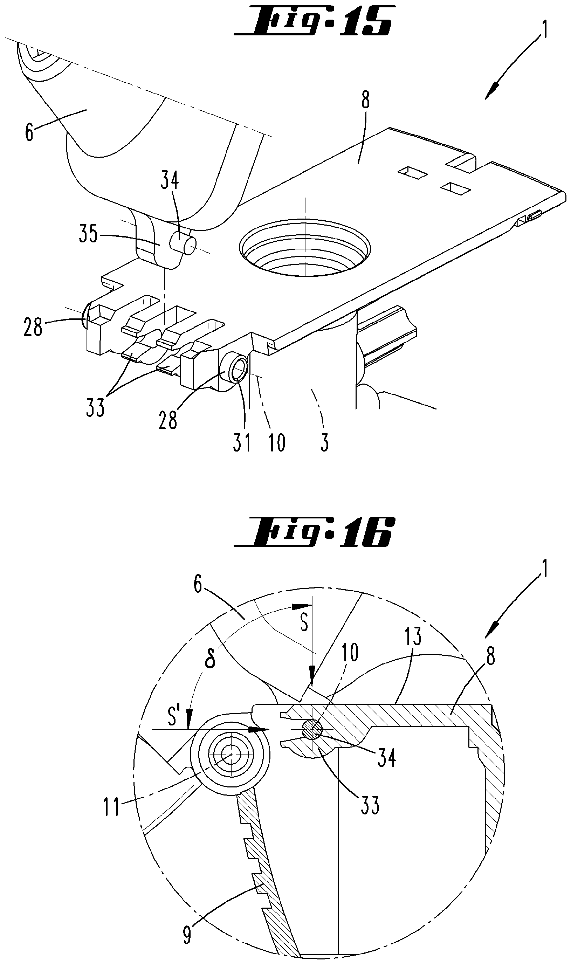

Zur Erreichung der jeweiligen Steckverbindung ist jeweils eine Steckrichtung S, S' vorgegeben. Wie insbesondere aus den Darstellungen in den Figuren 14 und 16 zu erkennen, schließen die Steckrichtung S zum Erreichen der Steckverbindung zwischen dem Verschlussdeckel 8 und dem Gehäuse 9 und die Steckrichtung S' zum Erreichen der Steckverbindung zwischen dem Mundstück 6 und dem Verschlussdeckel 8 projiziert in eine Ebene senkrecht zu der geometrischen Verschwenkachse 10 (wie dies in den Figuren 14 und 16 dargestellt ist) in Umfangsrichtung zu der geometrischen Verschwenkachse 10 einen spitzen bis hin zu rechten Winkel δ zueinander ein (insbesondere mit Bezug auf die dargestellte Nutzungsstellung des Verschlussdeckels 8).

### Liste der Bezugszeichen

| | | | |
|---|---|---|---|
| 1 | Vorrichtung | 29 | Steckausformung |
| 2 | Kapsel | 30 | Steckausformungsschenkel |
| 3 | Kapselaufnahme | 31 | Wandung |
| 4 | Nadel | 32 | Bereich |
| 5 | Handhabe | 33 | Steckausformung |
| 6 | Mundstück | 34 | Achszapfen |
| 7 | Verschlusskappe | 35 | Ausleger |
| 8 | Verschlussdeckel | 36 | Sichtfenster |
| 9 | Gehäuse | | |
| 10 | Verschwenkachse | E | Ebene |
| 11 | Verschwenkachse | D | Abmessung Kapselaufnahme |
| 12 | Randkante | K | Außenkontur |
| 13 | Oberkante | K' | Außenkontur |
| 14 | Lagerungsausformung | R | Einführungsrichtung |
| 15 | Verschwenkachse | S | Steckrichtung |
| 16 | Löcher | S' | Steckrichtung |
| 17 | Löcher | V | Vertikale |
| 18 | Löcher | | |
| 19 | Topfboden | α | spitzer Winkel |
| 20 | Topfwandung | β | spitzer Winkel |
| 21 | Wulst | γ | spitzer Winkel |
| 22 | Topfwandungsabschnitt | 6 | Winkel |
| 23 | Topfwandungsabschnitt | | |
| 24 | kragender Abschnitt | | |
| 25 | Rippen | | |
| 26 | Nut | | |
| 27 | Siebteil | | |
| 28 | Achszapfen | | |

## Patentansprüche

1. Vorrichtung (1) zum Inhalieren pulverförmiger Substanzen enthalten in Kapseln (2), mit einem Gehäuse (9), einer Kapselaufnahme (3), einem Verschlussdeckel (8) und einem Mundstück (6), wobei eine zum Überfangen zugleich des Mundstücks (6), des Verschlussdeckels (8) und der Kapselaufnahme (3) ausgebildete Verschlusskappe (7) vorgesehen ist, wobei weiter die Verschlusskappe (7), das Mundstück (6) und der Verschlussdeckel (8) verschwenkbar anscharniert sind, wobei zwei unterschiedliche, aber gleichgerichtet verlaufende Verschwenkachsen ausgebildet sind, eine erste Verschwenkachse (10) und eine zweite Verschwenkachse (11), die, bezogen auf eine Seitenansicht, in welcher sich geometrische Achsen der Verschwenkachsen (10, 11) punktförmig abbilden, nebeneinander und auf einer selben Seite der Kapselaufnahme (3) angeordnet sind, wobei weiter in einem Benutzungszustand der Vorrichtung die erste Verschwenkachse (10) näher an der Kapselaufnahme (3) angeordnet ist, der Verschlussdeckel (8) und das Mundstück (6) um die erste Verschwenkachse (10) verschwenkbar sind und allein die Verschlusskappe (7) um die zweite Verschwenkachse (11) verschwenkbar ist, **dadurch gekennzeichnet, dass** die Verschlusskappe (7), das Mundstück (6) und der Verschlussdeckel (8) jeweils um eine fest an dem Gehäuse (9) angebrachte Verschwenkachse (10, 11) verschwenkbar anscharniert sind und dass beide Verschwenkachsen (10, 11) relativ zueinander unbeweglich an dem Gehäuse (9) ausgebildet sind.

2. Vorrichtung nach Anspruch 1, **dadurch gekennzeichnet, dass** nur zwei Verschwenkachsen, die erste Verschwenkachse 10 und die zweite Verschwenkachse 11, ausgebildet sind.

3. Vorrichtung nach Anspruch 1, **dadurch gekennzeichnet, dass** an dem Verschlussdeckel (8) eine weitere Verschwenkachse (15) ausgebildet ist, die zusammen mit dem Verschlussdeckel (8) verschwenkbar ist.

4. Vorrichtung nach Anspruch 3, **dadurch gekennzeichnet, dass** um die weitere, dritte Verschwenkachse (15) allein das Mundstück (6) verschwenkbar ist.

5. Vorrichtung nach einem der vorhergehenden Ansprüche, **dadurch gekennzeichnet, dass** das Mundstück und die Verschlusskappe (7) bezogen auf die Seitenansicht, in welcher sich die geometrischen Achsen der Verschwenkachsen (10, 11) punktförmig abbilden, sich in einer am weitesten geöffneten Stellung bezüglich ihrer Außenkonturen (K, K') ohne Überdeckung zueinander erstrecken.

6. Vorrichtung nach einem der vorhergehenden Ansprüche, **dadurch gekennzeichnet, dass** das Mundstück (6) und der Verschlussdeckel (8) zu einer gemeinsamen Verschwenkung miteinander verrastbar sind.

7. Vorrichtung nach einem der vorhergehenden Ansprüche, **dadurch gekennzeichnet, dass** die Kapselaufnahme (3) und der Verschlussdeckel (8) zu einer gemeinsamen Verschwenkung miteinander verbunden sind.

8. Vorrichtung nach einem der vorhergehenden Ansprüche, **dadurch gekennzeichnet, dass** der Verschlussdeckel (8) mit dem Gehäuse (9) steckverbunden ist.

9. Vorrichtung nach einem der vorhergehenden Ansprüche, **dadurch gekennzeichnet, dass** das Mundstück (6) mit dem Gehäuse (9) steckverbunden ist.

10. Vorrichtung nach einem der vorhergehenden Ansprüche, **dadurch gekennzeichnet, dass** das Mundstück (6) mit dem Verschlussdeckel (8) steckverbunden ist.

11. Vorrichtung nach einem der vorhergehenden Ansprüche, **dadurch gekennzeichnet, dass** der Verschlussdeckel (8) angeformte Achszapfen (28) aufweist, zur Steckaufnahme in Steckausformungen (29) des Gehäuses (9) oder dass das Gehäuse (9) angeformete Achszapfen (28) aufweist, zur Steckaufnahme in Steckausformungen (29) des Verschlussdeckels (8).

12. Vorrichtung nach Anspruch 11, **dadurch gekennzeichnet, dass** die Steckausformungen (29) des Gehäuses (9) im Nutzungszustand der Vorrichtung (1) entgegen einer beim Einsteckvorgang sich ergebenden Verformungstendenz von einem Bereich (32) des Verschlussdeckels (8) umfasst sind.

13. Vorrichtung nach einem der Ansprüche 11 oder 12, **dadurch gekennzeichnet, dass** der Achszapfen (28) in einem Querschnitt mit einer kreisringförmigen Wandung (31) gebildet ist.

14. Vorrichtung nach einem der vorhergehenden Ansprüche, **dadurch gekennzeichnet, dass** der Verschlussdeckel (8) eine Steckausformung (33) für einen Achszapfen (34) des Mundstücks (6) aufweist oder dass das Mundstück (6) eine Steckausformung (33) für einen Achszapfen (34) des Verschlussdeckels (8) aufweist.

15. Vorrichtung nach einem der Ansprüche 11 bis 14, **dadurch gekennzeichnet, dass** die Steckrichtungen (S, S') zum Erreichen der Steckverbindungen zwischen dem Verschlussdeckel (8) und dem Gehäuse (9) einerseits und dem Mundstück (6) und dem Verschlussdeckel (8) andererseits projiziert in eine Ebene senkrecht zu der geometrischen Verschwenkachse (10) des Verschlussdeckels (8) in Umfangsrichtung zu der geometrischen Verschwenkachse (10) einen spitzen oder rechten Winkel (6) zueinander einschließen.

## Claims

1. A device (1) for inhaling powdery substances contained in capsules (2), comprising a housing (9), a capsule receiver (3), a closing cover (8), and a mouthpiece (6), wherein a closing cap (7) formed to simultaneously cover the mouthpiece (6), the closing cover (8), and the capsule receiver (3) is provided, wherein the closing cap (7), the mouthpiece (6), and the closing cover (8) are further hinged in a pivotable manner, wherein two pivot axes, which are different but run in the same direction, are formed, a first pivot axis (10) and a second pivot axis (11), which, with regard to a side view, in which geometric axes of the pivot axes (10, 11) are represented in the form of points, are arranged next to one another and on a same side of the capsule receiver (3), wherein in a usage state of the device the first pivot axis (10) is further arranged closer to the capsule receiver (3), the closing cover (8) and the mouthpiece (6) can be pivoted about the first pivot axis (10), and only the closing cap (7) can be pivoted about the second pivot axis (11), **characterized in that** the closing cap (7), the mouthpiece (6), and the closing cover (8) are in each case hinged so as to be pivotable about a pivot axis (10, 11), which is fixedly attached to the housing (9), and that both pivot axes (10, 11) are formed on the housing (9) so as to be immovable relative to one another.

2. The device according to claim 1, **characterized in that** only two pivot axes, the first pivot axis (10) and the second pivot axis (11), are formed.

3. The device according to claim 1, **characterized in that** a further pivot axis (15), which can be pivoted together with the closing cover (8), is formed on the closing cover (8).

4. The device according to claim 3, **characterized in that** only the mouthpiece (6) can be pivoted about the further, third pivot axis (15).

5. The device according to one of preceding claims, **characterized in that**, with regard to the side view, in which the geometric axes of the pivot axes (10, 11) are represented in the form of points, the mouthpiece and the closing cap (7) extend to one another without overlap with regard to their outer contours (K, K') in a most open position.

6. The device according to one of the preceding claims, **characterized in that** the mouthpiece (6) and the closing cover (8) can be locked with one another for a joint pivoting.

7. The device according to one of the preceding claims, **characterized in that** the capsule receiver (3) and the closing cover (8) are connected to one another for a joint pivoting.

8. The device according to one of the preceding claims, **characterized in that** the closing cover (8) is plugconnected to the housing (9).

9. The device according to one of the preceding claims, **characterized in that** the mouthpiece (6) is plugconnected to the housing (9).

10. The device according to one of the preceding claims, **characterized in that** the mouthpiece (6) is plugconnected to the closing cover (8).

11. The device according to one of the preceding claims, **characterized in that** the closing cover (8) has integrally molded axle journals (28), for the plug reception in plug moldings (29) of the housing (9), or that the housing (9) has integrally molded axle journals (28) for the plug reception in plug moldings (29) of the closing cover (8).

12. The device according to claim 11, **characterized in that** in contrast to a deformation tendency resulting during the insertion process, the plug moldings (29) of the housing (9) in the use state of the device (1) are encompassed by a region (32) of the closing cover (8).

13. The device according to one of claims 11 or 12, **characterized in that** in a cross section, the axle journal (28) is formed comprising an annular wall (31).

14. The device according to one of the preceding claims, **characterized in that** the closing cover (8) has a plug molding (33) for an axle journal (34) of the mouthpiece (6) or that the mouthpiece (6) has a plug molding (33) for an axle journal (34) of the closing cover (8).

15. The device according to one of claims 11 to 14, **characterized in that** the plug directions (S, S') for obtaining the plug connections between the closing cover (8) and the housing (9) on the one hand, and the mouthpiece (6) and the closing cover (8) on the other hand, draw an acute or right angle (δ) to one another, projected into a plane perpendicular to the geometric pivot axis (10) of the closing cover (8) in the circumferential direction to the geometric pivot axis (10) .

## Revendications

1. Dispositif (1) pour l'inhalation de substances pulvérulentes contenues dans des capsules (2), avec un boîtier (9), un réceptacle de capsule (3), un couvercle de fermeture (8) et un embout (6), dans lequel est prévu un capuchon de fermeture (7) formé pour recouvrir à la fois l'embout (6), le couvercle de fermeture (8) et le réceptacle de capsule (3), dans lequel en outre le capuchon de fermeture (7), l'embout (6) et le couvercle de fermeture (8) sont articulés de manière pivotante, dans lequel deux axes de pivotement différents mais orientés dans la même direction sont formés, un premier axe de pivotement (10) et un deuxième axe de pivotement (11) qui, par rapport à une vue de côté dans laquelle des axes géométriques des axes de pivotement (10, 11) se représentent sous forme ponctuelle, sont agencés l'un à côté de l'autre et sur un même côté du réceptacle de capsule (3), dans lequel en outre le premier axe de pivotement (10) est agencé plus près du réceptacle de capsule (3) dans un état d'utilisation du dispositif, le couvercle de fermeture (8) et l'embout (6) peuvent pivoter autour du premier axe de pivotement (10) et seul le capuchon de fermeture (7) peut pivoter autour du deuxième axe de pivotement (11), **caractérisé en ce que** le capuchon de fermeture (7), l'embout (6) et le couvercle de fermeture (8) sont chacun articulés de manière pivotante autour d'un axe de pivotement (10, 11) monté fixement au boîtier (9) et **en ce que** les deux axes de pivotement (10, 11) sont formés joints au boîtier (9) de manière immobile l'un par rapport à l'autre

2. Dispositif selon la revendication 1, **caractérisé en ce que** seuls deux axes de pivotement sont formés, le premier axe de pivotement (10) et le deuxième axe de pivotement (11).

3. Dispositif selon la revendication 1, **caractérisé en ce qu'**un autre axe de pivotement (15) est formé joint au couvercle de fermeture (8), lequel peut pivoter conjointement avec le couvercle de fermeture (8).

4. Dispositif selon la revendication 3, **caractérisé en ce que** seul l'embout (6) peut pivoter autour de l'autre, troisième axe de pivotement (15).

5. Dispositif selon l'une des revendications précédentes, **caractérisé en ce que** par rapport à la vue de côté dans laquelle les axes géométriques des axes de pivotement (10, 11) se représentent sous forme ponctuelle, l'embout et le capuchon de fermeture (7) s'étendent sans recouvrement l'un par rapport à l'autre dans une position la plus ouverte par rapport à leurs contours extérieurs (K, K').

6. Dispositif selon l'une des revendications précédentes, **caractérisé en ce que** l'embout (6) et le couvercle de fermeture (8) peuvent être encliquetés l'un avec l'autre pour un pivotement conjoint.

7. Dispositif selon l'une des revendications précédentes, **caractérisé en ce que** le réceptacle de capsule (3) et le couvercle de fermeture (8) sont reliés entre eux pour un pivotement conjoint.

8. Dispositif selon l'une des revendications précédentes, **caractérisé en ce que** le couvercle de fermeture (8) est lié au boîtier (9) par emboîtement.

9. Dispositif selon l'une quelconque des revendications précédentes, **caractérisé en ce que** l'embout (6) est lié au boîtier (9) par emboitement.

10. Dispositif selon l'une des revendications précédentes, **caractérisé en ce que** l'embout (6) est lié au couvercle de fermeture (8) par emboitement.

11. Dispositif selon l'une quelconque des revendications précédentes, **caractérisé en ce que** le couvercle de fermeture (8) présente des tourillons (28) qui y sont formés pour être reçus par emboîtement dans des conformations d'emboîtement (29) du boîtier (9) ou **en ce que** le boîtier (9) présente des tourillons (28) qui y sont formés pour être reçus par emboîtement dans des conformations d'emboîtement (29) du couvercle de fermeture (8).

12. Dispositif selon la revendication 11, **caractérisé en ce que**, dans l'état d'utilisation du dispositif (1), les conformations d'emboîtement (29) du boîtier (9) sont entourées par une zone (32) du couvercle de fermeture (8), à l'encontre d'une tendance à la déformation résultant de l'opération d'emboîtement.

13. Dispositif selon l'une des revendications 11 ou 12, **caractérisé en ce que** l'axe (28) est formé dans une section transversale avec une paroi (31) en forme d'anneau circulaire.

14. Dispositif selon l'une quelconque des revendications précédentes, **caractérisé en ce que** le couvercle de fermeture (8) présente une conformation d'emboîtement (33) pour un tourillon (34) de l'embout (6) ou **en ce que** l'embout (6) présente une conformation d'emboîtement (33) pour un tourillon (34) du couvercle de fermeture (8).

15. Dispositif selon l'une des revendications 11 à 14, **caractérisé en ce que** les directions d'emboîtement (S, S') pour obtenir les liaisons par emboîtement entre le couvercle de fermeture (8) et le boîtier (9) d'une part et l'embout (6) et le couvercle de fermeture (8) d'autre part, projetées dans un plan perpendiculaire à l'axe géométrique de pivotement (10) du couvercle de fermeture (8), forment entre elles un angle aigu ou droit (6) dans la direction circonférentielle par rapport à l'axe géométrique de pivotement (10).
